# EUROPÄISCHE PATENTSCHRIFT

(11) **EP 4 011 272 B1**
(45) Veröffentlichungstag und Bekanntmachung des Hinweises auf die Patenterteilung: **14.05.2025**
(21) Anmeldenummer: 21210415.2
(22) Anmeldetag: 25.11.2021
(51) Int. Cl.: A61B 1/12, A61B 1/015, A61B 1/018, A61B 1/00

(54) **ENDOSKOP MIT REINIGBARER ROTATIONSTROMMEL**
ENDOSCOPE WITH CLEANABLE ROTATING DRUM
ENDOSCOPE POURVU DE TAMBOUR ROTATIF POUVANT ÊTRE NETTOYÉ

(30) Priorität: 09.12.2020 DE 102020132773
(43) Veröffentlichungstag der Anmeldung: 15.06.2022
(73) Patentinhaber: Karl Storz SE & Co. KG, 78532 Tuttlingen (DE)
(72) Erfinder: Schwarz, Peter, 78532 Tuttlingen (DE)

(56) Entgegenhaltungen:
- US-A1- 2003 032 863
- US-A1- 2009 088 631
- US-A1- 2015 216 402
- US-A1- 2017 224 197
- US-A1- 2019 381 290

## Beschreibung

Die vorliegende Erfindung betrifft ein Endoskop mit einer reinigbaren Rotationstrommel für medizinische Anwendungen nach dem Oberbegriff des Anspruchs 1.

Die WO 2017/040692 A1 zeigt ein derartiges Endoskop mit einer kugelförmigen Rotationstrommel an einem langerstreckten starren Schaftrohr, insbesondere für die Verwendung als ein Einweginstrument, das in einer Lagergabel an einem distalen Ende des starren Schaftrohres drehbar gelagert ist, und mit einer Steuerleitung im Inneren des starren Schaftrohres rotiert werden kann. Die Rotationstrommel weist im Inneren ein Abbildungssystem auf und ist mit elektronischen Elementen im Inneren des starren Schaftrohres verbunden, wobei gleichzeitig eine Flüssigkeit durch das Innere des starren Schaftrohres geleitet werden kann. Das Abbildungssystem kann gereinigt werden, indem das Abbildungssystem mit einer Blickrichtung, insbesondere einer Abbildungsoptik des Abbildungssystems, in das Innere des starren Schaftrohres geschwenkt wird. In dieser geschwenkten Reinigungsstellung der Rotationstrommel kann das Abbildungssystem mittels der Flüssigkeit im Inneren des starren Schaftrohres umspült und gereinigt werden.

Die US 2019/381290 A1 zeigt längliche chirurgische Vorrichtungen mit einem zentralen Lumen und einer aufklappbaren Struktur an einem distalen Ende der Vorrichtung. An einem proximalen Ende ist eine Bedieneinheit für die aufklappbare Struktur angeordnet. Zusätzlich bildet die Vorrichtung einen Fluidanschluss aus, der mit dem zentralen Lumen und der aufklappbaren Struktur in einer Fluidverbindung steht.

Die US 2003/032863 A1 offenbart ein Endoskop, umfassend eine Bildgebungseinheit, die am distalen Ende eines Schaftes positioniert ist; einen Schwenkmechanismus, der mechanisch mit der Bildgebungseinheit gekoppelt ist; und einen Betätigungsmechanismus, der sich durch den Durchgang des Schaftes erstreckt und mit dem Schwenkmechanismus gekoppelt ist. Die Bildgebungseinheit umfasst eine Objektivlinse, einen Bildgeber und eine Lichtquelle.

Die US 2015/216402 A1 zeigt ein Laparoskop mit einem länglichen Schaftrohr, wobei eine Objektivlinse mit einer transparenten Kugel an einer distalen Seite des Schaftrohrs angeordnet ist. Die Kugel ist drehbar im distalen Ende des Schaftrohrs gelagert, so dass ein Teil der Kugel aus dem Rohr herausragt. Ein ringförmiger Wischer ist am distalen Ende des Schaftrohrs befestigt, um in Umfangsrichtung mit der Kugel in Eingriff zu kommen. In dem Schaftrohr sind Mittel zum Drehen der Kugel angeordnet, wobei der Wischer Schmutz und Flüssigkeiten von der Oberfläche der Kugel entfernt, wenn sich die Kugel dreht.

Die US 2017/224197 A1 offenbart eine Endoskophülle mit Positioniervorrichtungen und Tropfrückhaltemerkmalen, wobei die Hülle so konfiguriert ist, dass sie ein Endoskop ganz oder teilweise aufnimmt und eine Fluidleitung bereitstellt, wenn das Endoskop darin eingeführt ist. Die Positionierungsvorrichtungen sind in einem distalen Endbereich der Endoskophülle angeordnet, so dass das Endoskop in der Endoskophülle gesichert ist. Die Tropfenrückhaltemerkmale sind derart konfiguriert, um einen Bereich zu erzeugen, der durch die Endoskophülle und das Endoskop begrenzt ist, um ein Flüssigkeitsvolumen durch Kapillarwirkung zurückzuhalten.

Die US 2009/088631 A1 offenbart einen Katheter mit einem ablenkbaren Element, das sich an einem distalen Ende des Katheters befindet. Das ablenkbare Element kann ein Ultraschallwandlerarray umfassen. Der Katheter kann ein Lumen umfassen, das sich von einem proximalen Ende des Katheters zu dem distalen Ende erstreckt. Das ablenkbare Element kann schwenkartig über einen Bogen von mindestens 90 Grad selektiv ablenkbar sein.

Ein Schaft oder üblicherweise ein Schaftrohr des Endoskops kann starr oder flexibel oder abschnittsweise flexibel ausgeführt sein. Im Folgenden wird, wenn nicht genauer beschrieben, unter dem Schaftrohr ein starres und/oder flexibles Schaftrohr verstanden.

In der Reinigungsstellung ist also das Abbildungssystem dem zu beobachtenden Operationsbereich abgewandt und eine Verfolgung eines Objekts in dem Operationsbereich muss für die Reinigung unterbrochen werden. Dadurch kann es zu einer unerwünschten Unterbrechung und Beeinträchtigung einer medizinischen, insbesondere chirurgischen, Operation kommen.

Der vorliegenden Erfindung liegt die Aufgabe zugrunde, ein Endoskop vorzuschlagen, das bei Vermeidung der aus dem Stand der Technik bekannten Probleme eine Reinigung während der Verwendung des Endoskops ermöglicht, ohne dabei die Blickrichtung, zum Beispiel von einem Operationsbereich, zu verändern und gleichzeitig eine Temperierung, insbesondere Kühlung, des Abbildungssystems zu verbessern.

Diese Aufgabe wird hinsichtlich des Endoskops mit den Merkmalen des unabhängigen Anspruchs 1 gelöst.

Vorteilhafte Ausführungsformen sind Gegenstand der Unteransprüche.

Erfindungsgemäß wird ein Endoskop mit einer Rotationstrommel oder einem Rotationsmodul und einem langerstreckten starren und/oder flexiblen Schaftrohr, das an einem distalen Ende mittels einer Lagergabel die Rotationstrommel um eine erste Rotationsachse rotierbar lagert und wobei in der Rotationstrommel ein optisches Abbildungssystem angeordnet ist. Dabei weist die Lagergabel zumindest eine erste Fluidleitung und zumindest eine Düse auf, um das Abbildungssystem zu reinigen und/oder zu kühlen.

Unter der Rotationstrommel oder dem Rotationsmodul wird im Rahmen der Erfindung allgemein eine Vorrichtung zur Aufnahme eines Abbildungssystems verstanden, vorzugsweise handelt es sich dabei um eine schwenkbare oder rotierbare Vorrichtung an einem distalen Ende des Schaftrohres und wird deshalb im Rahmen der Erfindung vorzugsweise als Rotationstrommel benannt, wobei es sich in einer bevorzugten Form um ein trommelförmiges Rotationsmodul handelt.

Unter dem Fluid kann im Rahmen der Erfindung vorzugsweise eine Flüssigkeit und/oder Gas verstanden werden, wobei für einen medizinischen Einsatz vorzugsweise ein steriles Fluid, wie aufbereiteter Sauerstoff als Luft/Gas und eine physiologische Salzlösung als Flüssigkeit verwendet wird.

Als Düsen werden Austrittslöcher aus dem Fluidkanal durch eine Wand der Lagergabel verstanden, die vorzugsweise einen kleineren Querschnitt als die Fluidleitung aufweisen, um das Fluid mit einer höheren Geschwindigkeit als in der Fluidleitung auf das zu reinigende Objekt zu sprühen.

Dabei hat die Erfindung erstaunlicherweise erkannt, dass durch die Verwendung der zumindest einen ersten Fluidleitung in der Lagergabel Düsen mit einem geringen Abstand zu der Rotationstrommel und ebenfalls dem Abbildungssystem angeordnet werden können. Dadurch kann in besonders effektiverweise die Rotationstrommel mit einem Fluid umspült werden, um das Abbildungssystem zu reinigen und/oder zu kühlen/temperieren und/oder eine Anströmung von verunreinigten Fluid abzulenken. So ist es nicht erforderlich einen gesamten Operationsbereich mit Fluid zu spülen, sondern es kann bereits mit geringem Fluideinsatz das Abbildungssystem lokal gereinigt werden, insbesondere auch mit einer Luftströmung für einen Einsatz in einem trockenen Operationsbereich. Durch den geringen Abstand und vorzugsweise die Ausrichtung der zumindest einen Düse auf das Abbildungssystem kann eine Reinigung in einer bestimmten Blickrichtung oder einem Blickbereich erfolgen, ohne zum Beispiel die Rotationstrommel mit der Blickrichtung in die Richtung des Inneren des Schaftrohres schwenken zu müssen.

Ein Kühlen der Rotationstrommel ist insbesondere aufgrund einer Wärmeentwicklung im Betrieb der elektronischen Komponenten des Abbildungssystems mit elektronischem Bildaufnehmer und/oder Beleuchtungseinrichtung erforderlich.

Allgemein kann die Fluidleitung für eine Temperierung der Rotationstrommel verwendet werden. So kann es alternativ zu einer Kühlung auch erforderlich sein das Abbildungssystem vor einer Operation auf die zu erwartende Umgebungstemperatur in einem Operationsbereich vorzuheizen.

Üblicherweise ist ein Anstieg der Umgebungstemperatur zu erwarten und ein Vorheizen kann ein Beschlagen der Abbildungsoptik des Abbildungssystems vorbeugen. Insbesondere um ein Beschlagen der Abbildungsoptik in einem trockenen Operationsbereich zu verhindern, ist als Fluid eine Luft- oder Gasströmung ausreichend.

Die Erfindung hat weiterhin erkannt, dass durch die Fluidleitung innerhalb der Lagergabel auch ein Fluid aus dem Operationsbereich gesaugt werden kann. Dadurch kann ein das Abbildungssystem potentiell verunreinigendes Fluid abgesaugt werden. Insbesondere für diesen Fall können die Düsen auch größer als der Querschnitt der Fluidleitung ausgebildet sein, um vorzugsweise ein Verstopfen/Blockieren durch ein verunreinigtes Fluid, zum Beispiel durch Gewebereste, der Düsen zu reduzieren.

Erfindungsgemäß steht die Lagergabel zu einer Position eines Blickfelds des Abbildungssystems in der Blickrichtung des Abbildungssystems, vorzugsweise entlang einer Längsachse des Schaftrohrs, zumindest teilweise vor, wobei zumindest eine erste Düse an einem distalen Ende der Lagergabel angeordnet ist, um das Blickfeld des Abbildungssystems mit dem Fluid zu reinigen. Insbesondere dadurch kann das Blickfeld des Abbildungssystems direkt, ohne ein Umspülen, mittels eines Sprühstrahls/Fluidströmung aus der zumindest einen ersten Düse gereinigt werden, um so auch durch ein mechanisches Abwaschen die Reinigungswirkung zu verbessern. Bevorzugt ist die Lagergabel derart verlängert, dass sich der Außendurchmesser des distalen Endes des Endoskops nicht vergrößert.

Durch die vorstehende Lagergabel lässt sich in vorteilhafterweise auch die Rotationstrommel vor einer Beschädigung schützen.

In einer besonders vorteilhaften Ausführungsform der Erfindung ist die zumindest eine erste Düse derart ausgerichtet ist, dass eine Fluidströmung mit einem Winkel zwischen 120° und 240°, bevorzugt 180°, bezogen auf die Längsachse des Schaftrohrs aus der zumindest einen ersten Düse austritt. Dadurch kann ein Abbildungssystem besonders mit einer 0° Blickrichtung während der Führung des Endoskops in den Operationsbereich gereinigt werden.

Weiterhin ist bevorzugt, dass zumindest eine zweite Düse im Bereich einer Lagerposition der Rotationstrommel angeordnet ist und auf die zumindest eine Seitenwand der Rotationstrommel gerichtet ist. Die zumindest eine zweite Düse ist bevorzugt zusätzlich zu der zumindest einen ersten Düse an dem distalen Ende der Lagergabel vorgesehen. Dadurch kann die angeströmte Außenfläche der Rotationstrommel vergrößert werden und eine Wärmeübertragung verbessert werden, um die Rotationstrommel zu temperieren, insbesondere die elektronischen Komponenten innerhalb der Rotationstrommel zu kühlen. Des Weiteren kann dadurch der üblicherweise für eine Fluidströmung schwer zugängliche Bereich zwischen der Lagergabel und der Seitenwand der Rotationstrommel gespült und einer Verunreinigung und einem Klemmen der Rotationstrommel vorgebeugt werden.

In einer Weiterbildung ist die zumindest eine Seitenwand der Rotationstrommel vorzugsweise mit einer die Oberfläche der zumindest einen Seitenwand vergrößernden Beschichtung mit Mikrostrukturen versehen. Dadurch kann die Wärmeübertragung der Seitenwand zu einem Kühlfluid weiter verbessert werden.

Weiter bevorzugt ist das Fluid mittels einer zumindest einen zweiten Fluidleitung innerhalb des Schaftrohres in die zumindest eine erste Fluidleitung führbar.

Vorzugsweise ist die zumindest eine zweite Fluidleitung Teil eines Arbeitskanals, der modular bevorzugt eine Luftleitung und/oder eine Flüssigkeitszufuhrleitung und eine Flüssigkeitsabfuhrleitung aufweist. Insbesondere da das Abbildungssystem mit Versorgungsleitung nicht innerhalb des Schaftrohres angeordnet ist, können insbesondere mehrere Kanäle, wie die Luftleitung zusammen mit der Flüssigkeitszufuhrleitung und der Flüssigkeitsabfuhrleitung innerhalb des Schaftrohres vorgesehen sein. Eine Art Überschaft um das Schaftrohr zur Fluidleitung, der den Außendurchmesser des Schaftrohrs vergrößert, ist vorteilhafterweise nicht notwendig. Die Funktionalität der zumindest einen zweiten Fluidleitung kann die Optikreinigung einer gedrehten Rotationstrommel und/oder ein rückseitiges Kühlen der Rotationstrommel und/oder Spülen und Insufflieren eines Fluids in einen Operationsbereich umfassen.

Vorteilhafterweise wird die Luftleitung in einem trockenen Operationsbereich eingesetzt, um die Rotationstrommel nach einer Optikreinigung zu trocknen und/oder die Rotationstrommel und der elektronischen Komponenten des Abbildungssystems zu kühlen. Die Optikreinigung des Endoskops kann auch in einem trockenen Operationsbereich in einer Reinigungsstellung stattfinden, in der die Rotationstrommel insbesondere derart rotiert ist, dass das Blickfeld auf das distale Ende des Schaftrohrs und den Arbeitskanal ausgerichtet ist. In dieser Reinigungsstellung kann das Abbildungssystem mit einer Spülflüssigkeit aus der Flüssigkeitszufuhrleitung gespült werden, wobei mit der Flüssigkeitsabfuhrleitung die Spülflüssigkeit entfernbar ist und so ein Austritt an Spülflüssigkeit in einen trockenen Operationsbereich minimiert werden kann. Vorzugsweise mit der Luftleitung lässt sich die Spülflüssigkeit von dem Abbildungssystem entfernen, um eine Störung des Blickfelds des Abbildungssystems durch Tropfenbildung zu verhindern. Weiter bevorzugt kann die Flüssigkeitszufuhrleitung und vorzugsweise auch die Luftleitung als Spritzdüse ausgebildet sein, um die Reinigungswirkung zu verbessern. Für einen wässrigen oder nassen Operationsbereich oder Umgebung des Endoskops ist vorzugsweise die Flüssigkeitszufuhrleitung und die Flüssigkeitsabfuhrleitung in dem Arbeitskanal angeordnet, um die Rotationstrommel rückseitig zu umspülen und zu temperieren, sowie den Operationsbereich zu spülen. Für die Spülung des medizinischen Operationsbereichs, ist bevorzugt vorgesehen, dass das Schaftrohr an einem distalen Ende umfangsseitig Löcher und/oder eine, vorzugsweise schlitzförmige, Öffnung des Arbeitskanals aufweist, um die zumindest eine zweite Fluidleitung, bevorzugt zumindest die Flüssigkeitsabfuhrleitung, mit dem Operationsbereich zu verbinden und um ein Spülfluid aus dem Operationsbereich zu entfernen. Insbesondere ist das Schaftrohr hinter der Rotationstrommel geöffnet, um einen freien Austritt des Spülfluids, insbesondere des Spülwassers, zu gewährleisten.

Gemäß einer weiteren Ausführungsform weist die Lagergabel einen ersten und einen zweiten Schenkel auf, die die Rotationstrommel dazwischen schwenkbar lagert, wobei in jedem der Schenkel zumindest eine erste Fluidleitung und zumindest eine Düse ausgebildet ist. Dadurch ist die Rotationstrommel aus zwei Richtungen von der Seite reinigbar und temperierbar.

Vorzugsweise ist zumindest eine Düse in einem ersten Schenkel der Lagergabel mit einer Fluidleitung als Flüssigkeitsleitung, insbesondere Wasserleitung, und zumindest eine Düse in einem zweiten Schenkel der Lagergabel mit einer Fluidleitung als Gas-/Luftleitung verbunden. Mit der Flüssigkeitsleitung lässt sich eine Verschmutzung des Abbildungssystems, insbesondere der Abbildungsoptik, spülen. Die Gas-/Luftleitung kann besonders für eine trockene Operationsumgebung vorzugsweise durch eine dauerhafte Anströmung des Abbildungssystems eine Verschmutzung durch Staub oder Beschlagen vorgebeugt werden.

Gemäß einer bevorzugten Weiterbildung weist die Lagergabel der Rotationstrommel ein abgerundetes distales Ende auf. Mit diesem abgerundeten Ende lässt sich das Endoskop mit einem geringen Verletzungsrisiko in einen Operationsbereich führen.

Es kann gemäß der Lehre der Erfindung vorgesehen sein, dass die Rotationstrommel mittels zumindest einer Steuerleitung um eine erste Rotationsachse schwenkbar ist und die zumindest eine Steuerleitung der Rotationstrommel auf einer Außenseite/Außenfläche des Schaftrohres, bevorzugt einen Arbeitskanal im Inneren des Schaftrohres und den Innendurchmesser des Schaftrohres nicht einschränkend, verläuft und mit zumindest einem Hebelabstand zu der ersten Rotationsachse an der Rotationstrommel befestigt ist.

Vorzugsweise ist die zumindest eine Steuerleitung bevorzugt auf den äußeren Umfang der Rotationstrommel entlang zumindest einer Aufwickelkurve aufwickelbar. Dadurch kann die Steuermechanik besonders einfach ausgeführt werden und es werden keine wartungsintensive oder störungsanfällige Zahnräder oder Übersetzungen und deren Lagerung benötigt.

Weiter bevorzugt ist, dass die zumindest eine Steuerleitung der Rotationstrommel als eine Versorgungsleitung für das Abbildungssystem, vorzugsweise als Flexleiterplatte oder als ein Kabel, für elektronische Schaltkreise im Inneren der Rotationstrommel ausgeführt ist. Dadurch kann neben der elektrischen oder elektronischen Ansteuerung der Rotationstrommel auch die mechanische Steuerung, insbesondere Verschwenkung durch die Steuerleitung realisiert werden.

Die Versorgungsleitung kann elektronische Schaltkreise insbesondere einer elektronisch verstellbaren Abbildungsoptik und/oder eines elektronischen Bildaufnehmers oder Sensors des Abbildungssystems und/oder einer Beleuchtungseinrichtung, vorzugsweise mit LEDs, aufweisen. Durch die gleichzeitige Verwendung der Versorgungsleitung als Steuerleitung kann besonders vorteilhaft auf eine weitere Steuerleitung für die Rotation der Rotationstrommel verzichtet werden.

Alternativ oder zusätzlich zu der zumindest einen zweiten Fluidleitung innerhalb des Schaftrohres kann die zumindest eine erste Fluidleitung auch mit einer weiteren außenliegenden Fluidleitung verbunden sein. Diese außenliegende Fluidleitung ist vorzugsweise zusammen mit der zumindest einen Steuerleitung der Rotationstrommel auf der Außenseite/Außenfläche des Schaftrohres geführt. Dadurch ist ein Arbeitskanal im Inneren des Schaftrohres für andere Funktionen verwendbar.

Diese außenliegende Fluidleitung kann alternativ auch auf die Rotationstrommel zusammen mit der zumindest einen Steuerleitung auf die Rotationstrommel aufwickelbar sein, die dazu ausgebildet ist einen Operationsbereich mit einem Fluid zu spülen.

Besonders bevorzugt ist die zumindest eine Steuerleitung in einer Aussparung, vorzugsweise mit einem Schutzkragen in der Außenfläche des Schaftrohres geführt, wobei die Aussparung parallel zu der Längsachse des Schaftrohres verläuft. Dadurch ist die zumindest eine Steuerleitung platzsparend und formschlüssig auf der Außenseite des Schaftrohres geführt, um einerseits die zumindest eine Steuerleitung vor mechanischen äußeren Einflüssen zu schützen und um andererseits einem Verletzungsrisiko oder Einklemmen von Gewebe während einer Operation vorzubeugen.

Für die Steuerung der Rotationstrommel sind an der Rotationstrommel vorzugsweise Rückstellmittel angeordnet, bevorzugt eine Drehfeder in einer Lagerstelle der Rotationstrommel. Dabei wirkt die Drehfeder einem mechanischen Moment der auf Zug beanspruchten zumindest einen Steuerleitung entgegen, um eine geschwenkte Rotationstrommel in eine Ausgangsposition zu versetzen. Die Drehfeder wirkt also vorzugsweise derart einer Bedienung der zumindest einen Steuerleitung entgegen, dass die zumindest eine Steuerleitung für eine Rotation der Rotationstrommel nicht mit einer Druckkraft beaufschlagt werden muss und um eine flexible Steuerleitung verwenden zu können.

Alternativ oder zusätzlich kann die Rotationstrommel durch eine weitere Steuerleitung auf der gegenüberliegenden Seite zurückführbar sein und/oder mit einer formstabilen Steuerleitung oder einem Art Bowdenzug oder einem Art Steuerstab oder Zahnstange auch mittels einer Druckkraft bedienbar sein.

Weiter alternativ kann die weitere Steuerleitung vorzugsweise als ein Draht ausgebildet sein, wobei ein Rückstellmittel, vorzugsweise eine Feder, insbesondere eine Schraubenfeder, bevorzugt in einem Handgriff zur Rückstellung der Rotationstrommel befestigt ist.

Vorzugsweise ist der Arbeitskanal innerhalb des Schaftrohres zur Aufnahme zumindest eines Instruments ausgebildet und/oder weist zumindest ein Instrument auf, um in dem Operationsbereich und insbesondere in dem Blickfeld des Abbildungssystems bevorzugt medizinische Operationen durchführen zu können. Dabei weist das zumindest eine Instrument an einem distalen Ende ein steuerbares Werkzeug, zum Beispiel ein scherenartiges Schneidewerkzeug oder Stanzwerkzeug, auf. Die Aufnahme des zumindest einen Instruments kann zusätzlich oder alternativ zu der zumindest einen zweiten Fluidleitung innerhalb des Schaftrohres vorgesehen sein. Durch die außenliegende Steuerleitung und insbesondere die außenliegende Versorgung des Abbildungssystems kann der relativ große Arbeitskanal für zumindest ein Instrument und gleichzeitig eine Vielzahl an zweiten Fluidleitungen verwendet werden.

In einer Weiterbildung, ist das Schaftrohr an einem oberen Abschnitt geöffnet, wobei die zumindest eine Steuerleitung an einem gegenüberliegenden unteren Abschnitt geführt ist, um vorzugsweise ein flexibles Instrument in eine Richtung senkrecht zu der Längsachse des Schaftrohrs und in den geöffneten oberen Abschnitt zu verstellen/biegen. Dadurch kann das Instrument, insbesondere die funktionellen Teile und Werkzeuge an dem distalen Ende des zumindest einen Instruments, aus dem Schaftrohr heraus in einen Operationsbereich gebracht werden und gleichzeitig kann der Operationsbereich mit dem Abbildungssystem innerhalb der Rotationstrommel verfolgt werden.

In einer weiteren Ausführungsform ist die Lagergabel um eine zweite Rotationsachse rotierbar, wobei die zumindest eine Düse vorzugsweise mittels einer flexiblen Schlauchverbindung mit der zumindest einen zweiten Fluidleitung und/oder einer außenliegenden weiteren Fluidleitung entlang des Schaftrohres verbunden ist.

Besonders für die Verwendung des Endoskops mit zumindest einem Instrument ist vorzugsweise vorgesehen, dass die Lagergabel schwenkbar um eine zweite Rotationsachse an dem Schaftrohr befestigt ist und der Arbeitskanal in einem geschwenkten Zustand der Lagergabel entlang der Längsachse geöffnet ist, um insbesondere ein Instrument aus dem Arbeitskanal in den Operationsbereich zu führen, wobei die Lagergabel vorzugsweise mit einer weiteren Steuerleitung steuerbar ist. Die weitere Steuerleitung ist vorzugsweise ebenfalls auf der Außenseite des Schaftrohres geführt, um den Arbeitskanal im Inneren des Schaftrohres nicht einzuschränken. Mit dieser Ausführungsform ist insbesondere ein starres Instrument parallel zu der Längsachse des Schaftrohrs in den Operationsbereich führbar. Des Weiteren ist mittels einer Rotation der Rotationstrommel um die zweite Rotationsachse ein vorzugsweise flexibles Instrument in eine Richtung senkrecht zu der Längsachse des Schaftrohrs biegbar. Neben der Führung des zumindest einen Instruments ermöglicht die schwenkbare Lagergabel vorteilhafterweise ein erweitertes Blickfeld des Abbildungssystems, auch zum Beispiel hinter Hindernisse, Ecken oder einen Blick entlang der Außenseite des Schaftrohrs. So ist zum Beispiel auch die Funktion der Führung der zumindest einen Steuerleitung entlang des Schaftrohres mittels des Abbildungssystems überprüfbar. Weiterhin kann mit dem geöffneten Arbeitskanal ein Spülen oder Absaugen von einem Fluid aus einem Operationsbereich begünstigt werden.

Alternativ oder zusätzlich kann die Lagergabel auch mittels eines flexiblen oder elastisch verformbaren Elements mit dem Schaftrohr verbunden sein, wobei das flexible Element eine passive Rückstellkraft ausbildet, um die Lagergabel um die zweite Rotationsachse zu schwenken und den Arbeitskanal für das Instrument zu öffnen.

Vorzugsweise beträgt der Außendurchmesser des Schaftrohres 3mm bis 6mm, wobei bevorzugt die Rotationstrommel diesen Außendurchmesser nicht übersteigt. Solche Schaftrohre sind für eine Vielzahl nicht invasiver medizinischer Operationen geeignet.

In einer weiter bevorzugten Ausführungsform sind mehrere Steuerleitungen auf unterschiedlichen Aufwickelkurven entlang des Umfangs der Rotationstrommel aufwickelbar, insbesondere mit einem unterschiedlichen Abstand zu der Rotationsachse, um bei gleichem Verstellweg der Steuerleitung eine unterschiedliche Drehverstellung der Rotationstrommel auszuführen. Insbesondere der Verstellweg parallel zu der Längsachse des Schaftrohres und die unterschiedliche Drehverstellung kann zu vordefinierten Blickrichtungen des Abbildungssystems oder zu unterschiedlichen Rotationsgeschwindigkeiten führen, wobei bei gleichem Verstellweg der Steuerleitung die Drehverstellung der Rotationstrommel mit kleiner werdendem Abstand zu der ersten Drehachse der Rotationstrommel abnimmt. Auch die für die Drehverstellung notwendige Zugkraft kann durch die unterschiedlichen Abstände eingestellt werden.

Besonders bevorzugt sind die unterschiedlichen Aufwickelkurven stufenweise entlang der ersten Rotationsachse auf dem Umfang der Rotationstrommel angeordnet, wobei vorzugsweise die Aufwickelkurven mit kleiner werdendem Abstand zu der ersten Rotationsachse in Richtung der äußeren Seiten der Rotationstrommel angeordnet sind.

Die Offenbarung betrifft auch ein Verfahren zur Reinigung eines Abbildungssystems bevorzugt eines vorangehend beschriebenen Endoskops mit Rotationstrommel, wobei die Rotationstrommel zuerst in eine Blickrichtung, vorzugsweise in eine einem Schaftrohr abgewandten Richtung, geschwenkt wird, um zum Beispiel einen Operationsbereich zu untersuchen und anschließend dauerhaft oder zumindest zeitweise mit einem Fluidstrom aus zumindest einer Fluidleitung mit zumindest einer Düse innerhalb einer Lagergabel der Rotationstrommel beaufschlagt wird. Dadurch kann das Abbildungssystem gereinigt werden, ohne einen Operationsvorgang zu unterbrechen oder zu beeinflussen.

Weitere Vorteile und Einzelheiten der Erfindung ergeben sich aus der nachfolgenden Beschreibung bevorzugter Ausführungsformen der Erfindung sowie anhand von lediglich schematischen Zeichnungen.

Es zeigen:
- Fig. 1a:: eine Seitenansicht auf ein distales Ende eines Endoskops mit Rotationstrommel und Lagergabel mit Fluidleitung,
- Fig. 1b:: einen Längsschnitt der Rotationstrommel gemäß der Fig. 1a,
- Fig. 2a:: eine Seitenansicht des Endoskops gemäß der Fig. 1a mit ersten und zweiten Düsen in der Lagergabel,
- Fig. 2b:: eine Draufsicht auf das Endoskop gemäß der Fig. 2a entlang einer Längsachse,
- Fig. 3a:: eine Seitenansicht des Endoskops gemäß der Fig. 1a mit drehbar gelagerter Lagergabel,
- Fig. 3b:: eine Seitenansicht des Endoskops gemäß der Fig. 3a mit einem Instrument und geschwenkter Lagergabel.

Gleiche Elemente beziehungsweise Elemente mit gleicher Funktion sind in den Figuren mit den gleichen Bezugsziffern versehen.

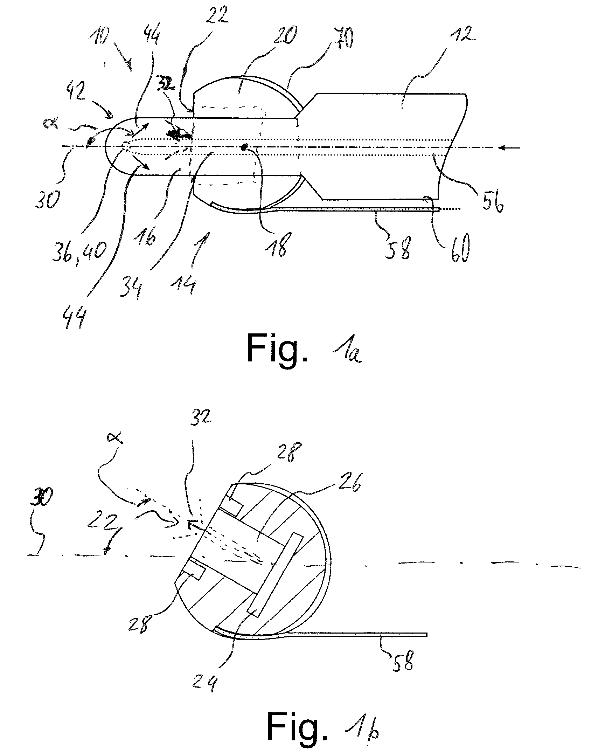

In der Fig. 1a ist ein Endoskop 10 mit einer Rotationstrommel 20 an einem distalen Ende 14 eines langerstreckten starren Schaftrohr 12 dargestellt, wobei die Rotationstrommel 20 mittels einer Lagergabel 16 an dem distalen Ende 14 des Schaftrohrs 12 um eine erste Rotationsachse 18 rotierbar gelagert ist.

Wie in der Fig. 1b im Detail gezeigt, ist in der Rotationstrommel 20 ein optisches Abbildungssystem 22 angeordnet, welches vorzugsweise einen elektronischen Bildaufnehmer 24, eine Abbildungsoptik 26 und eine Beleuchtungseinrichtung 28 umfasst.

Eine Blickrichtung 32 des Abbildungssystems 22 ist mit einem Winkel α zu der Längsachse 30 des Schaftrohrs 12 schwenkbar, wobei die Lagergabel 16 die Blickrichtung 32 der Abbildungsoptik 26 nicht einschränkt. Durch Schwenken der Rotationstrommel 20 und dadurch der Blickrichtung 32 kann ein Winkel α von bevorzugt über 130° abgedeckt werden. Durch Rotation des Schaftrohres 12 um die Längsachse 30 kann der Beobachtungsbereich des Abbildungssystems 22 erweitert werden.

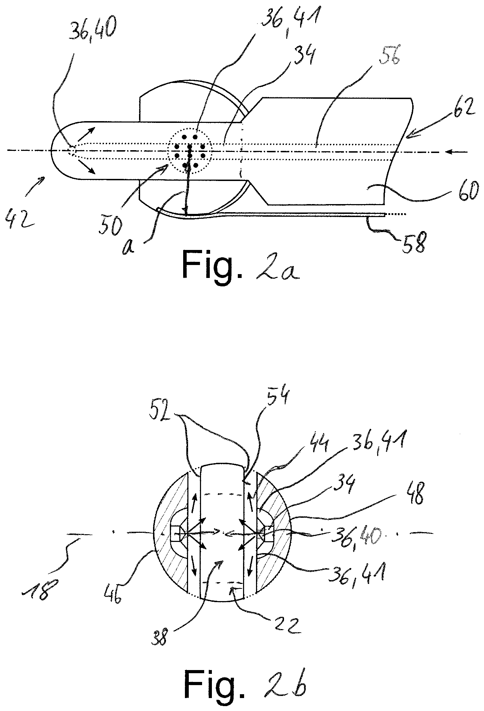

Wie in der Fig. 1a und der Fig. 2a gezeigt, weist die Lagergabel 16 zumindest eine erste Fluidleitung 34 und zumindest eine Düse 36 auf, um das Abbildungssystem 22 zu reinigen und/oder zu kühlen.

Die Lagergabel 16 ist vorzugsweise entlang der Längsachse 30 des Schaftrohrs 18 zu einer Position des Blickfelds 38 des Abbildungssystems 22 verlängert. Wie in der Fig. 1a dargestellt, ist zumindest eine erste Düse 36, 40 an einem distalen Ende 42 der Lagergabel 16 angeordnet, um das Blickfeld 38 des Abbildungssystems 22 mit einer Fluidströmung 44 zu reinigen. Bei einer Verlängerung der Lagergabel 16 entlang der Längsachse 30 ist das Blickfeld 38 bei einer Blickrichtung 32 mit einem Winkel α von 0° reinigbar. Besonders bevorzugt kann in dieser Stellung das Endoskop 10 zu einem Operationsbereich geführt werden, wobei gleichzeitig das Blickfeld 38 mit der zumindest einen ersten Düse 36, 40 reinigbar ist.

Vorzugsweise ist die zumindest eine erste Düse 26 derart ausgerichtet, dass die Fluidströmung 44 mit einem Winkel α zwischen 120° und 240°, bevorzugt 180°, bezogen auf die Längsachse 30 des Schaftrohrs 12, aus der zumindest einen ersten Düse 36, 40 austritt. Dadurch ist das Blickfeld 38 des Abbildungssystems 22 großflächig auch bei einem geringen Abstand zu der zumindest einen ersten Düse 36, 40 reinigbar. Insbesondere kann bei einem geringen Abstand die Reinigung durch ein Schwenken der Rotationstrommel 20 verbessert werden.

Alternativ kann die in der Fig. 1a gezeigt Fluidströmung 44 auch umgekehrt erfolgen, um ein Fluid oder ein potentiell verunreinigendes Medium vor dem Blickfeld 38 abzusaugen und um so einer Verunreinigung des Blickfelds 38 vorzubeugen.

Wie in der Fig. 2a dargestellt, ist vorzugsweise zumindest eine zweite Düse 36, 41, bevorzugt eine Vielzahl an zweiten Düsen 36, 41, im Bereich einer Lagerposition 50 der Rotationstrommel 20 angeordnet. Die zumindest eine zweite Düse 36, 41 ist gemäß der Fig. 2b auf die zumindest eine Seitenwand 52 der Rotationstrommel 20 gerichtet, um diese zu temperieren, insbesondere zu kühlen. Dabei ist in der Fig. 2b die Fluidströmung 44 auf die zumindest eine Seitenwand 52 der Rotationstrommel 20 und auf das Blickfeld 38 des Abbildungssystems 22 dargestellt. Das Fluid aus der zumindest einen zweiten Düse 36, 41 kann ein Gas und/oder eine Flüssigkeit sein, wobei eine Flüssigkeit die Wärmeübertragung verbessert und ein Gas vorzugsweise in einem trockenen Operationsbereich einsetzbar ist.

Besonders bevorzugt ist die zumindest eine Seitenwand 52 der Rotationstrommel 20 mit einer die Oberfläche der zumindest einen Seitenwand 52 vergrößernden Beschichtung mit nicht dargestellten Mikrostrukturen versehen, um die Wärmeübertragung weiter zu verbessern.

Bevorzugt ist gemäß der Fig. 1a oder der Fig. 2a ein Fluid mittels einer zumindest einen zweiten Fluidleitung 56 innerhalb des Schaftrohres 12 in die zumindest eine erste Fluidleitung 34 führbar. Dabei ist die zumindest eine zweite Fluidleitung 56 vorzugsweise in einem Arbeitskanal 62 des Schaftrohres 12 angeordnet.

Bevorzugt weist die Lagergabel 16 einen ersten und einen zweiten Schenkel 46, 48, gemäß der Fig. 2b, auf, um die Rotationstrommel 20 dazwischen schwenkbar zu lagern, wobei in jedem der Schenkel 46, 48 zumindest eine erste Fluidleitung 34 und zumindest eine Düse 36 ausgebildet ist.

Vorzugsweise ist wie in der Fig. 2a gezeigt, die zumindest eine erste Düse 36, 40 und die zumindest eine zweite Düse 36, 41 mit einer gemeinsamen ersten Fluidleitung 34 verbunden. Alternativ können auch eine Vielzahl an ersten Fluidleitungen 34 vorgesehen sein, um zum Beispiel die zumindest eine erste Düse 36, 40 und die zumindest eine zweite Düse 36, 41 mit unterschiedlichen Fluiden zu versorgen. So kann vorzugsweise die zumindest eine zweite Düse 36, 41 mit einer Flüssigkeit befüllt sein, um die Wärmeübertragung und Temperierung der Rotationstrommel 20 zu verbessern. Dabei kann vorzugsweise die zumindest eine erste Düse 36, 40 mit einem Gas oder Luft befüllt sein, um das Blickfeld 38 des Abbildungssystems 22 zu reinigen. Eine Vielzahl weiterer Kombinationen ist hier möglich, die insbesondere von einer Umgebungsbedingung in einem Operationsbereich abhängt. Zum Beispiel kann für einen trockenen Operationsbereich ausschließlich eine Gas oder Luftströmung bevorzugt sein und für einen nassen Operationsbereich ausschließlich eine Flüssigkeitsströmung.

Des Weiteren kann die Variation an ersten Fluidleitungen 34 auch auf die zwei Schenkel 46, 48 bezogen sein. So kann zumindest eine Düse 36 in einem ersten Schenkel 46 der Lagergabel 16 mit einer Flüssigkeitsleitung, insbesondere Wasserleitung, und zumindest eine Düse 36 in einem zweiten Schenkel 48 mit einer Gas-/Luftleitung verbunden sein.

Besonders bevorzugt weist die Lagergabel 16 der Rotationstrommel 20, gemäß der Fig. 1a oder Fig. 2a, ein abgerundetes distales Ende 42 auf.

Wie in der Fig. 1a oder der Fig. 2a dargestellt, ist die Rotationstrommel 20 vorzugsweise mittels einer Steuerleitung 58 um eine erste Rotationsachse 18 schwenkbar, wobei die Steuerleitung 58 auf einer Außenseite/Außenfläche 60 des Schaftrohres 12, bevorzugt den Arbeitskanal 62 im Inneren des Schaftrohres 12 und einen Innendurchmesser des Schaftrohres 12 nicht einschränkend verläuft. Die Steuerleitung 58 ist mit einem Hebelabstand a zu der ersten Rotationsachse 18 an der Rotationstrommel 20 befestigt. Bevorzugt ist die Rotationstrommel 20 durch eine Bewegung der Steuerleitung 58 parallel zu der Längsachse 30 des Schaftrohrs 12 rotierbar.

Die Rotationstrommel 20 ist, gemäß der Fig. 1a und Fig. 1b, vorzugsweise kugel- oder zylinderförmig ausgeführt, wobei die Rotationstrommel 20 bevorzugt senkrecht zu der Blickrichtung 32 des Abbildungssystems 22 abgeflacht ist, um in einem abgeflachten Bereich die Abbildungsoptik 26 und die Beleuchtungseinrichtung 28, vorzugsweise als zwei LEDs, aufzunehmen.

Wie in der Fig. 1b gezeigt, ist die Steuerleitung 58 vorzugsweise auf eine Aufwickelkurve 70 entlang des äußeren Umfangs der Rotationstrommel 20 aufwickelbar. Besonders bevorzugt ist die Steuerleitung 58 derart aufwickelbar, dass die Rotationstrommel 20 von einer Blickrichtung 32 entlang der Längsachse 30 des Schaftrohres 12 um zumindest einen Winkel α von 0° bis 180° drehbar ist und so die Blickrichtung 32 in das Innere des Schaftrohres 12 und einen Arbeitskanal 62 ausrichtbar ist. Für das Schwenken der Rotationstrommel 20 ist die Steuerleitung 58 zumindest teilweise flexibel ausgeführt, um diese auf die Aufwickelkurve 58 der Rotationstrommel 20 aufzuwickeln. Dabei kann die Aufwickelkurve 58 entlang des äußeren Umfangs vorzugsweise kreisförmig oder oval ausgeführt sein.

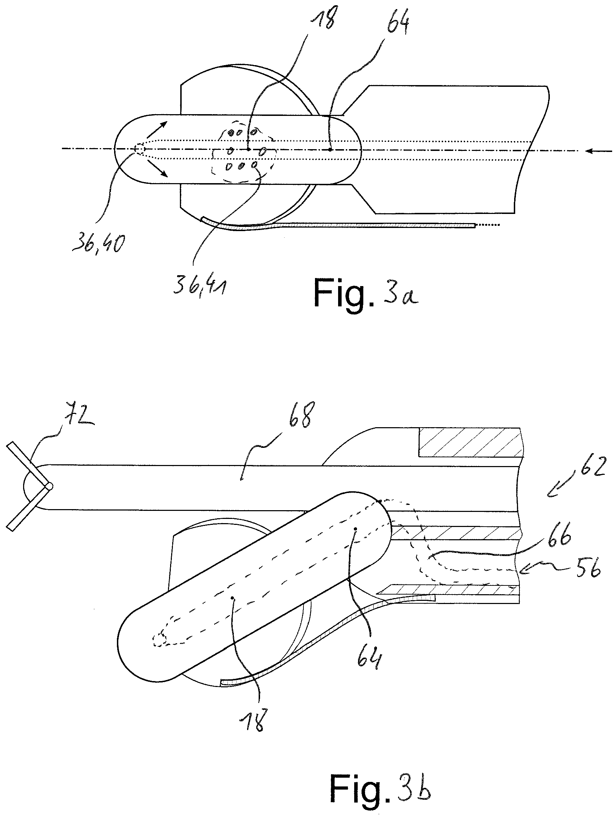

Wie in der Fig. 3a und der Fig. 3b gezeigt, ist die Lagergabel 16 in einer Weiterbildung um eine zweite Rotationsachse 64 rotierbar, wobei die zumindest eine Düse 36 vorzugsweise mittels einer flexiblen Schlauchverbindung 66 mit der zumindest einen zweiten Fluidleitung 56 und/oder einer außenliegenden weiteren Fluidleitung entlang des Schaftrohres 12 verbunden ist.

Vorzugsweise mit dieser rotierbaren Lagergabel 16 um eine zweite Rotationsachse 64 kann, wie in der Fig. 3b a dargestellt, der Arbeitskanal 62 dazu ausgebildet sein ein Instrument 68 aufzunehmen und/oder eines aufzuweisen. Dieses Instrument 68 kann an einem distalen Ende ein Werkzeug 72, zum Beispiel ein Schneidewerkzeug oder Stanzwerkzeug, aufweisen, das auf die jeweilige medizinische Operation anpassbar ist.

In einem geschwenkten Zustand der Lagergabel 16, gemäß der Fig. 3b, ist der Arbeitskanal 62 entlang der Längsachse 30 derart geöffnet ist, dass vorzugsweise ein starres Instrument 68 aus dem Arbeitskanal 62 in den Operationsbereich führbar ist. Die Lagergabel 16 ist vorzugsweise mit einer nicht dargestellten weiteren Steuerleitung oder einem anderen Bedienmechanismus steuerbar. Vorzugsweise einem Bedienmechanismus für flexible Schaftrohre, der zum Beispiel aus der US 2015/0359420 A1 bekannt ist.

Durch das Schwenken der Lagergabel 16 kann ebenfalls das Blickfeld 38 der Rotationstrommel 20 erweitert werden, um zum Beispiel auch ein von dem Schaftrohr 12 selbst abgedecktes Blickfeld 38 des Abbildungssystems 22 zu erfassen oder um hinter Ecken oder Hindernissee blicken zu können.

Kein Teil der Erfindung ist ein Verfahren zu Reinigung des Abbildungssystems 22 mit Rotationstrommel 20, wobei die Rotationstrommel 20 in eine Blickrichtung 32, gemäß der Fig. 1a geschwenkt wird, vorzugsweise mit einem Winkel α von 0°, um zum Beispiel einen Operationsbereich zu untersuchen. Anschließend wird das Abbildungssystem 22 dauerhaft oder zumindest zeitweise mit einem Fluidstrom 44 aus zumindest einer ersten Fluidleitung 34 mit zumindest einer Düse 36 innerhalb einer Lagergabel 16 der Rotationstrommel 20, bevorzugt aus der zumindest einen ersten Düse 36, 40, beaufschlagt.

Das soweit beschriebene Endoskop 10 kann in vielfältiger Art und Weise abgewandelt beziehungsweise modifiziert werden, ohne vom Erfindungsgedanken abzuweichen. So ist es zum Beispiel denkbar, dass der zu der Position des Abbildungssystems 22 verlängerte Teil der Lagergabel 16 körperfest mit der Rotationstrommel 20 mitrotierbar ist, um das Abbildungssystem unabhängig von der geschwenkten Stellung der Rotationstrommel 20 zu reinigen.

Des Weiteren ist es denkbar, die Reinigung und/oder Temperierung der Rotationstrommel 20 oder des Abbildungssystems 22 mit einem Regelkreis zu koppeln oder automatisiert auszuführen, um zum Beispiel eine bestimmte Temperatur einzustellen oder das Abbildungssystem 22 zu reinigen, sobald eine Verschmutzung vorzugsweise mit dem Bildaufnehmer 24 detektiert wird.

### Bezugszeichenliste

- 10: Endoskop
- 12: Schaftrohr
- 14: distale Ende des Schaftrohrs
- 16: Lagergabel
- 18: erste Rotationsachse
- 20: Rotationstrommel
- 22: Abbildungssystem
- 24: elektronischer Bildaufnehmer
- 26: Abbildungsoptik
- 28: Beleuchtungseinrichtung
- 30: Längsachse des Schaftrohrs
- 32: Blickrichtung der Abbildungsoptik
- 34: erste Fluidleitung
- 36: Düse
- 38: Blickfeld des Abbildungssystems
- 40: erste Düse
- 41: zweite Düse
- 42: distale Ende der Lagergabel
- 44: Fluidströmung
- 46: erster Schenkel der Lagergabel
- 48: zweiter Schenkel der Lagergabel
- 50: Lagerposition der Rotationstrommel
- 52: Seitenwand der Rotationstrommel
- 54: Oberfläche der Seitenwand
- 56: zweite Fluidleitung
- 58: Steuerleitung der Rotationstrommel
- 60: Außenseite des Schaftrohres
- 62: Arbeitskanal
- 64: zweite Rotationsachse
- 66: flexible Schlauchverbindung
- 68: Instrument
- 70: Aufwickelkurve
- 72: Werkzeug
- a: Hebelabstand
- α: Winkel zwischen der Blickrichtung und der Längsachse

## Patentansprüche

1. Endoskop (10) mit einer Rotationstrommel (20) und einem langerstreckten starren und/oder flexiblen Schaftrohr (12), das an einem distalen Ende (14) mittels einer Lagergabel (16) die Rotationstrommel (20) um eine erste Rotationsachse (18) rotierbar lagert und wobei in der Rotationstrommel (20) ein optisches Abbildungssystem (22) angeordnet ist,
**dadurch gekennzeichnet,**
**dass** die Lagergabel (16) zumindest eine erste Fluidleitung (34) und zumindest eine erste Düse (36) aufweist, um das Abbildungssystem (22) zu reinigen und/oder zu kühlen, wobei die Lagergabel (16) zu einer Position eines Blickfelds (38) des Abbildungssystems (22) in einer Blickrichtung (32) des Abbildungssystems (22), vorzugsweise entlang einer Längsachse (30) des Schaftrohrs (12), zumindest teilweise vorsteht und wobei die zumindest eine erste Düse (36, 40) an einem distalen Ende (42) der Lagergabel (16) angeordnet ist, um das Blickfeld (38) des Abbildungssystems (22) mit einem Fluid zu reinigen.

2. Endoskop nach dem Anspruch 1,
**dadurch gekennzeichnet,**
**dass** die zumindest eine erste Düse (36, 40) derart ausgerichtet ist, dass eine Fluidströmung (44) mit einem Winkel (α) zwischen 120° und 240°, bevorzugt 180°, bezogen auf die Längsachse (30) des Schaftrohrs (12) aus der zumindest einen ersten Düse (36, 40) austritt.

3. Endoskop nach dem Anspruch 1 oder 2,
**dadurch gekennzeichnet,**
**dass** zumindest eine zweite Düse (36, 41) im Bereich der Lagerposition (50) der Rotationstrommel (20) angeordnet ist und auf zumindest eine Seitenwand (52) der Rotationstrommel (20) gerichtet ist.

4. Endoskop nach einem der Ansprüche 1 bis 3,
**dadurch gekennzeichnet,**
**dass** zumindest eine Seitenwand (52) der Rotationstrommel (20) mit einer eine Oberfläche (54) der zumindest einen Seitenwand (52) vergrößernden Beschichtung mit Mikrostrukturen versehen ist.

5. Endoskop nach einem der Ansprüche 1 bis 4,
**dadurch gekennzeichnet,**
**dass** ein Fluid mittels einer zumindest einen zweiten Fluidleitung (56) innerhalb des Schaftrohres (12) in die zumindest eine erste Fluidleitung (34) führbar ist.

6. Endoskop nach einem der Ansprüche 1 bis 5,
**dadurch gekennzeichnet,**
**dass** die Lagergabel (16) einen ersten und einen zweiten Schenkel (46, 48) aufweist und die Rotationstrommel (20) dazwischen schwenkbar lagert, wobei in jedem der Schenkel (46, 48) zumindest eine erste Fluidleitung (34) und zumindest eine Düse (36) ausgebildet ist.

7. Endoskop nach einem der Ansprüche 1 bis 6,
**dadurch gekennzeichnet,**
**dass** die Lagergabel (16) der Rotationstrommel (20) ein abgerundetes distales Ende (42) aufweist.

8. Endoskop nach einem der Ansprüche 1 bis 7,
**dadurch gekennzeichnet,**
**dass** die Rotationstrommel (20) mittels zumindest einer Steuerleitung (58) um die erste Rotationsachse (18) schwenkbar ist und die zumindest eine Steuerleitung (58) der Rotationstrommel (20) auf einer Außenseite (60) des Schaftrohres (12), bevorzugt einen Arbeitskanal (62) im Inneren des Schaftrohres (12) und einen Innendurchmesser des Schaftrohres (12) nicht einschränkend, verläuft und mit zumindest einem Hebelabstand (a) zu der zumindest einen ersten Rotationsachse (18) an der Rotationstrommel (20) befestigt ist.

9. Endoskop nach einem der Ansprüche 1 bis 8,
**dadurch gekennzeichnet,**
**dass** die Lagergabel (16) um eine zweite Rotationsachse (64) rotierbar ist, wobei die zumindest eine Düse (36) vorzugsweise mittels einer flexiblen Schlauchverbindung (66) mit einer zumindest einen zweiten Fluidleitung (56) und/oder einer außenliegenden weiteren Fluidleitung entlang des Schaftrohres (12) verbunden ist.

## Claims

1. An endoscope (10) with a rotation drum (20) and an elongated rigid and/or flexible shaft tube (12), which at a distal end (14) supports the rotation drum (20) rotatably about a first axis of rotation (18) by means of a bearing fork (16) and wherein an optical imaging system (22) is arranged in the rotation drum (20),
**characterised in**
**that** the bearing fork (16) has at least one first fluid line (34) and at least one first nozzle (36) in order to clean and/or cool the imaging system (22), wherein the bearing fork (16) protrudes at least partially towards a position of a field of view (38) of the imaging system (22) in a viewing direction (32) of the imaging system (22), preferably along a longitudinal axis (30) of the shaft tube (12), and wherein the at least one first nozzle (36, 40) is arranged at a distal end (42) of the bearing fork (16) in order to clean the field of view (38) of the imaging system (22) with a fluid.

2. The endoscope according to claim 1,
**characterised in**
**that** the at least one first nozzle (36, 40) is aligned in such manner that a fluid flow (44) emerges from the at least one first nozzle (36, 40) at an angle (α) of between 120° and 240°, preferably 180°, relative to the longitudinal axis (30) of the shaft tube (12).

3. The endoscope according to claim 1 or 2,
**characterised in**
**that** at least one second nozzle (36, 41) is arranged in the region of the bearing position (50) of the rotation drum (20) and is directed towards at least one side wall (52) of the rotation drum (20).

4. The endoscope according to one of claims 1 to 3,
**characterised in**
**that** at least one side wall (52) of the rotation drum (20) is provided with a coating with microstructures which enlarges a top surface (54) of the at least one side wall (52).

5. The endoscope according to one of claims 1 to 4,
**characterised in**
**that** a fluid can be guided into the at least one first fluid line (34) by means of at least one second fluid line (56) within the shaft tube (12).

6. The endoscope according to one of claims 1 to 5,
**characterised in**
**that** the bearing fork (16) has a first and a second limb (46, 48) and the rotation drum (20) is pivotably supported therebetween, wherein at least one first fluid line (34) and at least one nozzle (36) is formed in each of the limbs (46, 48).

7. The endoscope according to one of claims 1 to 6,
**characterised in**
**that** the bearing fork (16) of the rotation drum (20) has a rounded distal end (42).

8. The endoscope according to one of claims 1 to 7,
**characterised in**
**that** the rotation drum (20) is pivotable about the first axis of rotation (18) by means of at least one control line (58) and the at least one control line (58) of the rotation drum (20) runs on an outer side (60) of the shaft tube (12), preferably not restricting a working channel (62) in the interior of the shaft tube (12) and an internal diameter of the shaft tube (12), and is fastened to the rotation drum (20) with at least one lever distance (a) from the at least one first axis of rotation (18).

9. The endoscope according to one of claims 1 to 8,
**characterised in**
**that** the bearing fork (16) is rotatable about a second axis of rotation (64), wherein the at least one nozzle (36) is preferably connected by means of a flexible hose connection (66) to at least one second fluid line (56) and/or an external further fluid line along the shaft tube (12).

## Revendications

1. Endoscope (10) comportant un tambour rotatif (20) et un tube de tige (12) rigide et/ou flexible allongé qui, à une extrémité distale (14), au moyen d'une fourche de palier (16), supporte le tambour rotatif (20) de manière à pouvoir tourner autour d'un premier axe de rotation (18) et dans lequel un système d'imagerie optique (22) est agencé dans le tambour rotatif (20),
**caractérisé,**
**en ce que** la fourche de palier (16) présente au moins une première conduite de fluide (34) et au moins une première buse (36) pour nettoyer et/ou refroidir le système d'imagerie (22), dans lequel la fourche de palier (16) fait saillie au moins partiellement vers une position d'un champ de vision (38) du système d'imagerie (22) dans une direction de vision (32) du système d'imagerie (22), de préférence le long d'un axe longitudinal (30) du tube de tige (12), et dans lequel l'au moins une première buse (36, 40) est agencée à une extrémité distale (42) de la fourche de palier (16) pour nettoyer le champ de vision (38) du système d'imagerie (22) avec un fluide.

2. Endoscope selon la revendication 1,
**caractérisé,**
**en ce que** l'au moins une première buse (36, 40) est orientée de telle sorte qu'un écoulement de fluide (44) sort de l'au moins une première buse (36, 40) avec un angle (α) compris entre 120 ° et 240 °, de préférence 180 °, par rapport à l'axe longitudinal (30) du tube de tige (12).

3. Endoscope selon la revendication 1 ou 2,
**caractérisé,**
**en ce qu'**au moins une seconde buse (36, 41) est agencée dans la zone de la position de palier (50) du tambour rotatif (20) et est dirigée vers au moins une paroi latérale (52) du tambour rotatif (20).

4. Endoscope selon l'une des revendications 1 à 3,
**caractérisé,**
**en ce qu'**au moins une paroi latérale (52) du tambour rotatif (20) est pourvue d'un revêtement à microstructures augmentant une surface (54) de l'au moins une paroi latérale (52).

5. Endoscope selon l'une des revendications 1 à 4,
**caractérisé,**
**en ce qu'**un fluide peut être guidé dans l'au moins une première conduite de fluide (34) au moyen d'au moins une seconde conduite de fluide (56) au sein du tube de tige (12).

6. Endoscope selon l'une des revendications 1 à 5,
**caractérisé,**
**en ce que** la fourche de palier (16) présente une première et une seconde branche (46, 48) et le tambour rotatif (20) est supporté de manière à pouvoir pivoter entre celles-ci, dans lequel au moins une première conduite de fluide (34) et au moins une buse (36) sont formées dans chacune des branches (46, 48).

7. Endoscope selon l'une des revendications 1 à 6,
**caractérisé,**
**en ce que** la fourche de palier (16) du tambour rotatif (20) présente une extrémité distale (42) arrondie.

8. Endoscope selon l'une des revendications 1 à 7,
**caractérisé,**
**en ce que** le tambour rotatif (20) peut pivoter autour du premier axe de rotation (18) au moyen d'au moins une conduite de commande (58) et l'au moins une conduite de commande (58) du tambour rotatif (20) s'étend sur un côté externe (60) du tube de tige (12), de préférence un canal de travail (62) à l'intérieur du tube de tige (12) et ne limitant pas un diamètre interne du tube de tige (12), et est fixé au tambour rotatif (20) avec au moins une distance de levier (a) par rapport à l'au moins un premier axe de rotation (18).

9. Endoscope selon l'une des revendications 1 à 8,
**caractérisé,**
**en ce que** la fourche de palier (16) peut tourner autour d'un second axe de rotation (64), dans lequel l'au moins une buse (36) est reliée de préférence au moyen d'un raccord de tuyau flexible (66) à au moins une seconde conduite de fluide (56) et/ou à une autre conduite de fluide placée à l'extérieur le long du tube de tige (12).
